(19) Europäisches Patentamt
European Patent Office
Office européen des brevets

(11) Publication number: **0 138 572**
**B1**

(12) # EUROPEAN PATENT SPECIFICATION

(45) Date of publication of patent specification: **25.07.90**

(51) Int. Cl.$^5$: **C 08 B 37/08,** A 61 K 31/73

(21) Application number: **84306914.7**

(22) Date of filing: **10.10.84**

(54) **Hyaluronic acid fractions having pharmaceutical activity, methods for preparation thereof, and pharmaceutical compositions containing the same.**

(30) Priority: **11.10.83 IT 4914383**
**09.10.84 IT 4897984**

(43) Date of publication of application:
**24.04.85 Bulletin 85/17**

(45) Publication of the grant of the patent:
**25.07.90 Bulletin 90/30**

(84) Designated Contracting States:
**AT DE GB IT NL SE**

(56) References cited:
**US-A-2 583 096**
**US-A-3 396 081**
**US-A-4 303 676**

**CHEM. & MOL. BIOL. OF THE INTERCELLULAR MATRIX, vol. 2, 1970, pages 703-732, E.A. Balazs; T.C. LAURENT: "Structure of hyaluronic acid"**

(73) Proprietor: **FIDIA S.p.A.**
**Via Ponte della Fabbrica 3-A**
**I-35031 Abano Terme (Padova) (IT)**

(72) Inventor: **della Valle, Francesco**
**Via Cerato 14**
**Padova (IT)**
Inventor: **Romeo, Aurelio**
**Viale Ippocrate 93**
**Rome (IT)**
Inventor: **Lorenzi, Silvana**
**Via Euganea 108**
**35100 Padova (IT)**

(74) Representative: **Pendlebury, Anthony et al**
**PAGE, WHITE & FARRER 54 Doughty Street**
**London WC1N 2LS (GB)**

(56) References cited:
**CHEMICAL ABSTRACTS, vol. 87, no. 21, 21st November 1977, page 170, no. 163114a, Columbus, Ohio, US; J.R.E. FRASER et al.: "Proteins retained with hyaluronic acid during ultrafiltration of synovial fluid" & CONNECT. TISSUE RES. 1977, 5(2), 61-5**

**EP 0 138 572 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

### Background And Field Of The Invention

This invention relates to specific molecular weight fractions of hyaluronic acid (hereinafter referred to as "HA") which have therapeutic applications and which are non-inflammatory when utilized. One of the HA fractions of this invention is useful for facilitating wound healing, while the second HA fraction is useful for intraocular use to substitute for endobulbar fluids or for intraarticular injection for use in treating damaged bone joints. The HA fractions have further been found to be useful as vehicles for ophthalmic drugs providing formulations compatible with the corneal epithelium and enhancing the activity of ophthalmic drugs.

Hyaluronic acid is a naturally occurring heteropolysaccharide consisting of alternating residues of D-glucuronic acid and N-acetyl-D-glucosamine. HA is a linear polymer of high molecular weight, generally up to about 8 to 13 million, and has been found in cell coats, the extracellular ground substance of connective tissues of vertebrates, in the synovial fluid in joints, in the endobulbar fluids of the eye, in human umbilical cord tissue, and in rooster combs.

Previous investigations on the use of HA are included in the work of Balazs, U.S. Patents Nos. 4303676 and 4141973. U.S. Patent No. 4303676 describes the use of certain viscoelastic compositions incorporating different sodium hyaluronate fractions, the first such fraction having a molecular weight of about 10,000 to 200,000 and a second such fraction having a molecular weight of about 10,000 to 200,000. U.S. Patent 4141973 is directed to a fraction of HA useful for replacing endobulbar fluids, as well as other therapeutic applications. This patent, however, is specifically directed to an HA fraction having an average molecular weight greater than about 750,000 and preferably greater than about 1,200,000. Balazs specifically teaches that fractions of HA having an average molecular weight of less than 750,000 are not therapeutically useful because of their inflammatory activity. These lower molecular weight fractions of HA are discarded by Balazs. However, this results in discarding about 90% of the total amount of available HA obtainable from the source tissues, resulting in a use of only a small amount (about 10%) of the available HA.

Neither of the above-mentioned Balazs U.S. patents describes hyaluronic acid fractions which are substantially free of hyaluronic acid having a molecular weight of less than 30,000.

Contrary to the teachings of Balazs, the present applicant has discovered that lower molecular weight fractions of HA do indeed have useful pharmaceutical activity. Thus, according to the present invention, about 80% of the HA obtainable from various sources is utilized. In particular, the present applicant has discovered one fraction of HA which is useful for stimulating wound healing, and a second fraction of HA which is useful for intraocular injections to substitute for the endobulbar liquids in the eye and for intraarticular injections as a treatment for damaged joints.

### Brief Description Of The Drawing

Figure 1 is a graph showing the different fractions of hyaluronic acid which have been identified by the present inventors.

### Detailed Description Of The Invention

As discussed above, previous investigations of HA, as exemplified by the Balazs patent, have been directed to utilizing high molecular weight fractions with an average molecular weight of greater than 750,000. The present applicant has isolated and characterized two new fractions of HA, one having a low molecular weight and another having a medium ranged average molecular weight, which are considered to be substantially pure as they are of a high degree of purity and do not have inflammatory activity. These new fractions of HA can be obtained from various connective tissues which contain extractable amounts of hyaluronic acid. The specific fractions of the invention are differentiated and separated according to the molecular filtration techniques.

The first fraction isolated by the applicant has been named HYALASTINE, and has an average molecular weight of from about 50,000 to about 100,000. This HYALASTINE fraction has been determined to be suitable for therapeutic, veterinary and human use because of its wound healing activity. The second fraction isolated by the applicant has been labeled HYALECTIN, and has an average molecular weight of about 500,000 to about 730,000. This HYALECTIN fraction is suitable for use in ocular surgery as a substitute for endobulbar liquids and for veterinary and human therapy in traumatic and degenerative diseases of the joints.

HYALASTINE can be administered either as an intradermal injection or it can be applied as a topical agent for wound healing. HYALECTIN, on the other hand, is suitable for intraocular and intraarticular injection.

The present applicant has made intensive studies on the various fractions of HA and, in a significantly more precise way than previously accomplished, has specifically determined the therapeutically useful fractions of HA and the inflammatory and non-useful fractions of HA. As a result of these studies the present applicant has identified and investigated two specific characteristics of HA fractions, namely cell mobilization activity and intrinsic viscosity. The wound healing process in animals is facilitated by cellular mobilization, and particularly the mobilization of fibroblasts. On the other hand, cellular mobilization or proliferation activity (i.e., mytosis) is to be avoided in cases of surgery inside the ocular globe. This is particularly true in operations to correct retinal detachment where an increased rate of heating may cause harmful affects.

The intrinsic viscosity is also an important parameter to be considered in determining the utility of a fraction of HA. A fraction having a high intrinsic viscosity is useful for surgical uses, in the therapy of diseases of the joints of a traumatic and degenerative nature, and for replacing endobulbar liquids. On the other hand, high viscosity is an undesirable characteristic for fractions to be utilized as drugs for facilitating wound healing. In fact, fractions to be utilized in wound healing should have low viscosity so as to be more easily used in practical application.

The HYALASTINE fraction identified by the present applicant has been determined to have good mobilization or cell proliferation activity, and low viscosity characteristics. Accordingly, HYALASTINE has the characteristics desirable for a material useful in promoting wound healing.

The same characteristics make the HYALASTINE fraction undesirable for use in intraocular or intraarticular injection treatments.

The HYALECTIN fraction identified by the applicant has been determined to have negligible cell mobilization or proliferation activity, while at the same time having high viscosity. These characteristics, therefore, make the HYALECTIN fraction useful for intraocular and intraarticular injection treatments. But, on the other hand, HYALECTIN is not useful for wound healing treatments since this fraction does not exhibit cell mobilization activity.

In isolating useful fractions of hyaluronic acid, it is also important to obtain those fractions which do not have inflammatory activity. The Balazs patent noted above teaches that in order to obtain hyaluronic acid fractions without inflammatory activity, fractions having an average molecular weight of more than 750,000 must be utilized exclusively. Thus, Balazs discards the fractions having less than 750,000 average molecular weight as being non-useful because of inflammatory activity. Contrary to the teachings of Balazs, the present applicant has found that the inflammatory activity attributed by Balazs to fractions having an average molecular weight of less than 750,000 is actually due to impurities having an average molecular weight of less than 30,000. Thus, the present invention provides a method which comprises a series of molecular filtration techniques associated with chemical methods by which the inflammatory fraction having a molecular weight of less than 30,000 can be eliminated.

By the methods of the invention, it is possible to obtain useful fractions of hyaluronic acid having no inflammatory activity which, when considered together, constitute a total yield of about 80% of the total hyaluronic acid available from the particular starting materials. This 80% yield of the available hyaluronic acid comprises a combined fraction which is a combination of the HYALECTIN and HYALASTINE fractions and which has an average molecular weight of from about 250,000 to about 350,000. More specifically, the HYALECTIN fraction is obtained in a yield of about 30% of the available HA and the HYALASTINE fraction is obtained in a yield of about 50% of the available HA from the starting tissues. This factor is an important improvement over the process of the Balazs patent discussed above in that the present inventors have discovered that significantly increased amounts of the available hyaluronic acid are pharmaceutically useful. By utilizing only the fraction having an average molecular weight of greater than 750,000, the Balazs process obtained a yield of only about 10% of the original hyaluronic acid available from animal organs, and discards about 90% of the available hyaluronic acid. Thus, according to the present invention, the usefulness of the total hyaluronic extract has been greatly increased.

A comparison of the relative yields of the extraction of various fractions of hyaluronic acid is presented below in Table 1.

TABLE 1

| Type of Hyaluronic Acid | g per 100 g of fresh tissue | % | Reference |
|---|---|---|---|
| Total hyaluronic acid from rooster combs | 0.8 | 100 | Swann D.A. 1968, Biochim, Biophys. Acta 156, 17-29 |
| HA (Balazs type) | 0.08 | 10 | U.S. Patent 4,141,973 |
| HYALECTIN + HYALASTINE | 0.6 | 80 | present invention |
| HYALECTIN | 0.2 | 30 | present invention |
| HYALASTINE | 0.4 | 50 | present invention |
| Inflammatory fraction | 0.16 | 20 | present invention |

Figure 1 graphically shows the different HA fractions which the present applicant has identified. The solid bell-shaped curve of Figure 1 represents the approximate distribution of the HA fractions available from a starting tissue. Area "B" in Figure 1 represents the HA fraction identified by Balazs in U.S. Patent 4,141,973 as being pharmaceutically useful. Areas "A", I and II in Figure 1 are the fractions identified by the presnt applicant, area A being the inflammatory fraction having an average molecular weight less than 30,000; area I being the HYALASTINE fraction; and area II being the HYALECTIN fraction. From this graph, it can be seen that the Balazs method discards the large majority of available extractable HA by eliminating the HA fractions having an average molecular weight of less than 750,000. The present invention, on the other hand, permits the pharmaceutical use of a large proportion of available HA since the low molecular weight hyaluronic acid fraction having an average molecular weight of less than 30,000 causes the inflammatory activity which previous investigators have noted with various extracts of HA.

The present applicant has discovered that a large percentage of available HA can, in fact, be utilized for therapeutic purposes if separated according to the teachings of this invention depending upon the particular therapeutic application. While the Balazs patent specifically teaches the use of only about 10% of the available HA, the present invention permits the use of about 80% of the available HA either in the form of the HYALASTINE fraction for wound healing applications, or in the form of the HYALECTIN fraction for intraocular and intraarticular applications, or in the form of a combined HYALASTINE and HYALECTIN fraction which can also be used for wound healing applications.

The chemical and physical characteristics of the identified fractions have also been investigated by the present applicant, and these characteristics are summarized in Table 2.

Methods Of Preparation

Example 1

Method for obtaining a mixture of HYALASTINE and HYALECTIN fractions without inflammatory activity:

Hen crests, either fresh or frozen (3000 g), are minced in a meat mincer and then carefully homogenized in a mechanical homogenizer. The resulting paste is placed in an AISI 316 stainless steel container or in a glass container with 10 volumes of anhydrous acetone. The entire content is then agitated for 6 hours at a speed of 50 g/minute and left to separate for 12 hours, after which the acetone is syphoned off and discarded.

This extraction process is repeated until the discarded acetone has reached the correct humidity level (Karl-Fischer method).

The resulting substance is then centrifuged and vacuum dried at a suitable temperature for 5—8 hours. With this process, approximately 500—600 g of dry powder is obtained from the hen crests.

300 g of the dry powder is then submitted to an enzymatic digestion process with papain (0.2 g) through a buffered aqueous medium with a phosphate buffer in the presence of a suitable quantity of cysteine hydrochloride. This mixture is then agitated for 24 hours at 60 g/minute at a constant temperature of 60—65°C. This whole mass is cooled to 25°C adding 60 g of Celite® and the agitation is maintained for an additional hour.

TABLE 2 - CHEMICAL AND PHYSICAL CHARACTERISTICS

| Fractions | M.W. | Dynamic viscosity at 20°C | Titer in hyaluronic acid of % dry powder | Content in proteins as bovine albumin | Content in sulphurated mucopolysaccharides |
|---|---|---|---|---|---|
| HYALASTINE + HYALECTIN | 250,000-350,000 | 100 mP.s. (conc. 1% w/v) | >96%[a] | <0.5% | < 1% |
| HYALASTINE | 50,000-100,000 | 600 mP.s (conc. 5% w/v) | >96% | <0.5% | < 1% |
| HYALECTIN | 500,000-730,000 | 170 mP.s (conc. 1% w/v) | >96% | <0.5% | <1% |

[a]Values presented represent the titer in HA after elimination in water. For example, a titer of 96% represents that, after elimination of water, the powder contains 4% impurities and 96% hyaluronic acid.

The resulting mixture is filtered until a clear liquid is obtained. This clear liquid then undergoes molecular ultrafiltration by means of membranes with a molecular exclusion limit of 30,000 to retain on the membrane those molecules with a molecular weight of greater than 30,000. Five to six original volumes are ultrafiltered and, at the same time, distilled water is continually added to the product. The addition of distilled water is suspended and the product is ultrafiltered until it is reduced to one-third of its original volume.

The residue liquid is rendered 0.1M by adding sodium chloride and the temperature is brought to 50°C. 45 g of cetylpyridinium chloride is added while the product is being agitated at 60 g/minute. This mixture is agitated for 60 minutes, after which 50 g of Celite® is added. Under agitation the temperature of the product is reduced to 25°C and the precipitate formed is collected by means of centrifugation. The precipitate thus obtained is suspended in a 0.01M solution of sodium chloride (5 liters) containing 0.05% cetylpyridinium chloride. It is agitated for a further 60 minutes at 50°C. The temperature is lowered to 25°C and the precipitate centrifuged.

The washing process is then repeated three times and the precipitate finally gathered into containers holding 3 liters of a 0.05M solution of sodium chloride containing 0.05% of cetylpyridinium chloride. This is agitated at 60 g/minute for 60 minutes and maintained at a constant temperature of 25°C for a period of 2 hours. The supernatant is eliminated by means of centrifugation.

The procedure is thus repeated several times with a 0.1M sodium chloride solution containing 0.05% of cetylpyridinium chloride. The mixture is centrifuged and the supernatant discarded. The precipitate is dispersed in a 0.30M sodium chloride solution containing 0.05% of cetylpyridinium chloride (3 l). The mixture is agitated and both the precipitate and the clear liquid are gathered. Extraction is repeated on the precipitate an additional 3 times, each time using 0.5 liters of the same aqueous solution.

Finally, the residue precipitate is eliminated and the clear liquids united in a single container. The temperature of the liquid is increased to 50°C while agitating. The liquid is then brought to 0.23M with sodium chloride. 1 g of cetylpyridinium chloride is added and agitation maintained for 12 hours. The mixture is cooled to 25°C then filtered, first through Celite® packs and then through a filter (1 μ).

The resultant mixture then undergoes a further molecular ultrafiltration through membranes with a molecular exclusion limit of 30,000, ultrafiltering 3 original volumes with the addition of a 0.33M sodium chloride solution. The addition of the sodium chloride solution is suspended and the volume of the liquid reduced to a quarter of its original volume.

The solution thus concentrated is precipitated under agitation (60 g/minute) at a temperature of 25°C with three volumes of ethanol (95%). The precipitate is collected by centrifugation and the supernatant discarded. The precipitate is dissolved in 1 liter of 0.1M sodium chloride solution and the precipitation procedure is repeated with three volumes of 95% ethanol.

The precipitate is gathered and washed, first with 75% ethanol (three times), then with absolute ethanol (three times) and thirdly with absolute acetone (three times).

The product thus obtained (HYALASTINE + HYALECTIN fraction) has an average molecular weight of from 250,000 to 350,000.

The hyaluronic acid yield is equal to 0.6% of the original fresh tissue.

### Example 2

Method for obtaining the HYALASTINE fraction from the mixture obtained by the method described in Example 1:

The mixture obtained by the method described in Example 1 is dissolved in pyrogen free distilled water in proportions of 10 mg of product in 1 ml in water. The solution thus obtained undergoes molecular ultrafilitration through membranes with a molecular exclusion limit of 200,000 with a concentration technique and without addition of water on the membrane. During the ultrafiltration process through membranes with an exclusion limit of 200,000, molecules with molecule weight greater than 200,000 will not pass, whereas smaller molecules will pass through the membrane along with the water. During the filtration process no water is added in the compartment above the membrane; therefore, the volume in this compartment will decrease, along with an increase in the concentration of molecules with M.W. over 200,000. It is then ultrafiltered until the volume on the membrane is reduced to 10% of the initial volume. Two volumes of pyrogen free bidistilled water are added and the solution is again ultrafilitered until the volume is reduced to one-third. The operation is repeated another two times.

The solution which passes through the membrane is brought to 1.0M with sodium chloride and then precipitated with four volumes of ethanol at 95%. The precipitate is washed three times with 75% ethanol and then vacuum dried.

The product thus obtained (HYALASTINE fraction) has an average molecular weight between 50,000 and 100,000.

The hyaluronic acid yield is equal to 0.4% of the original fresh tissue.

### Example 3

Method of obtaining HYALECTIN fraction:

The concentrated solution, gathered into a container from the ultrafiltration membrane with a molecular exclusion limit of 200,000 described in Example 2, is diluted with water until a solution

containing 5 mg/ml of hyaluronic acid is obtained, as determined by quantitative analysis based upon an assay of glucuronic acid.

The solution is brought to 0.1M in sodium chloride and then precipitated with 4 volumes of 95% ethanol. The precipitate is washed three times with 75% ethanol and then vacuum dried.

The product thus obtained (HYALECTIN fraction) has an average molecular weight between 500,000 and 730,000. This corresponds to a specific hyaluronic acid fraction of defined molecular chain length of about 2,500 and 3,500 saccharide units with a high degree of purity.

The hyaluronic acid yield is equal to 0.2% of the original fresh tissue.

## Evaluation Of Biological And Pharmaceutical Activity

1. Biological activity of cellular mobilization of the hyaluronic acid fractions:

The method, consisting of the determination of the detachment activity of fibroblasts in culture, was used as the method for evaluating the cell mobilization activity of the HA fractions.

Mouse BALB 3T3 cells were grown in Dulbecco's modified Eagle medium supplemented with 10% calf serum, penicillin (250 units/ml) and streptomycin (0.25 mg/ml), and were incubated in humidified 5% $CO_2$, 95% air at 37°C. For experimental purposes, cells were routinely inoculated in 60-mm diameter plastic tissue culture dishes ($6 \times 10^5$ cells/dish).

Confluent monolayers of 3T3 cells were decanted and fresh medium containing 2.0 mg/ml of different fractions of HA were added. At fixed intervals, the cell detachment was observed both microscopically and by counting the mobilized cells in a Coulter Counter.

Detachment assay:

To measure detachment kinetics, cells were inoculated into plastic dishes and allowed to grow for 24 hours. After this time, the culture medium was decanted and fresh medium containing 2.0 mg/ml of HA was added. Every 24 hours, two dishes, one each of test culture and of control culture, were decanted and both the cells in the supernatant an the attached cells were counted in a Coulter Counter.

Table 3 reports the results obtained with tests using the fractions obtained in the preparation Examples 1—3 above.

TABLE 3

Results of Mobilization Studies

| Fraction | Concentration (mg/ml) | No. of Detached Cells as Compared to Controls | % Effectiveness (as compared to control) |
|---|---|---|---|
| Control | | $2 \times 10^6$ | |
| HYALECTIN + HYALASTINE | 2 | $3.5 \times 10^6$ | 75 |
| HYALECTIN | 2 | $2.1 \times 10^6$ | 5 |
| HYALASTINE | 2 | $5 \times 10^6$ | 150 |

The data reported in Table 3 shows that the HYALASTINE fraction exhibits high cell mobilization activity making this fraction useful for wound healing applications. This cell mobilization activity of HYALASTINE will stimulate migration and proliferation of new cells when a pharmaceutical preparation of the fraction is applied to a damage tissue area.

The HYALECTIN fraction, on the other hand, exhibits very little cell mobilizatioon activity and would not, therefore, be useful for wound healing. HYALECTIN, however, because of its high average molecular weight and inherent viscosity, is useful for ocular and intraarticular injections, and the lack of appreciable cell mobilization activity is an important characteristic of the HYALECTIN fraction making it especially useful for intraocular and intraarticular injections.

2. Biological inflammatory activity and the hyaluronic acid fractions:

For this evaluation, the method of invasive cell count after intraocular administration in rabbits is utilized.

Method

Five (5) New Zealand or California rabbits, weighing about 2 kg and with ascertained perfect vision, are used for this test. The outer eye of the rabbits is checked for inflammatory processes at a macroscopic level and the inner eye is checked by ophthalmoscope. If the ocular fundus is clearly visible and normal, the test may proceed.

The selected animals undergo local anaesthetic by the instillation of a few drops of a suitable sterile anesthetic for ophthalmology; a few drops of atropine in ophthalmological solution are also instilled.

The test is carried out in sterile conditions. The ocular globe is made to protrude by means of pressure until it is possible to inject, through the sclera at about 5—6 mm from the limbus, at the center of the vitreal cavity, 100 μl of the solution, using a 26 G needle. The other eye is taken as a control. Two drops of antibiotic solution are instilled in the treated eye and the animals are then housed in single cages.

After 50—60 hours the test may proceed further. The eyes are checked in the same way as above for the selection of the animals. The animals are sacrificed with an intravenous injection of Pentothal. Then, firstly, the aqueous humor (about 0.2 ml) is gathered by means of an insulin syringe with a 26 G needle. The ocular globes are then enucleated, freed from all foreign matter, washed in saline, dried with bilbulous paper, and incised and opened in Petri plates, separating the main part of the vitreous humor and gathering it with a sterile syringe (about 0.7 ml). The vitreous humor is placed in small polyethylene test tubes and 50 μl of hyaluronidase (100 U NF/ml) is added. The mixture is then kept at a temperature of 37°C for about 3 hours in order to render the solution less viscous.

A leukocyte count is carried out under a microcope by phase contrast (120 ×) in a Burker chamber. A series of counts is effected on each sample, calculating the average values and expressing the result as the number of leukocytes per $mm^3$.

The test is considered positive when:

1) the eyes examined show no signs of suffering, and

2) the average number of leukocytes from at least 4 of the 5 treated eyes does not exceed 200 per $mm^3$ and the average number of leukocytes from each control eye does not exceed 50 per $mm^3$.

Table 4 reports the results obtained from this evaluation using the HA fractions obtained in preparation Examples 1—3 discussed above.

TABLE 4

Results of Inflammatory Activity Studies

| Fraction | No. of Invasive Cells |
|---|---|
| Control | 25 |
| HYALASTINE + HYALECTIN | 32 |
| HYALASTINE | 20 |
| HYALECTIN | 22 |
| Inflammatory Fraction (Avg. MW of 30,000) | 150 |
| Total Hyaluronic Acid from Rooster Combs (RF. Swann D.A. 1968, B.B.A. 156, 17-29) | 120 |

The results reported in Table 4 show that the HYALASTINE and HYALECTIN fractions exhibit no inflammatory activity above that for the control, and the combined HYALASTINE and HYALECTIN fraction showed only a negligible increase in inflammatory activity over the control. Accordingly, the HYALASTINE and HYALECTIN fractions are pharmaceutically useful without exhibiting undesirable inflammatory side effects. These results also further confirm the applicant's discovery that it is the HA fraction having a low average molecular weight of less than about 30,000 which is responsible for the inflammatory activity of HA preparations. Hyaluronic acid from rooster combs, prepared according to the method described in the literature by Swann (Swann D. A. 1968, B.B.A. *156*, 17—29) shows a remarkable inflammatory activity.

It has thus been shown that the HYALASTINE fraction having an average molecular weight of about 50,000 to 100,000 exhibits high cell mobilization activity and is, therefore, useful in wound healing applications without exhibiting undesirable inflammatory reactions. The HYALECTIN fraction, having an average molecular weight of about 500,000 to 730,000, has been shown to be useful for ocular and intraarticular injection due to its high molecular weight and inherent viscosity, and, at the same time, does not stimulate cell mobilization activity or inflammatory reactions which are undesirable side effects to be avoided in these pharmaceutical applications.

More specifically, a HYALASTINE fraction has been found to be useful as a wound healing preparation because of the following characteristics.

1. The preparation promotes a remarkably shortened healing time, as compared to conventional therapy, with rapid clearing of the affected area, regularization of ulcer edges, vigorous development of granulation tissue, activation of cellular migration of macrophages and fibroblasts and early epithelization.

2. The preparation promotes increased amenability to reconstructive surgery in the more severe cases.

3. The absence of keloid or retracting scar formation, with final reshaping of cicatricial tissue yielding good cosmetic and functional results.

The HYALASTINE preparation has been found to be useful for the treatment of various wounds including decubitus sores (bed sores) trophic ulcers, burns, indolent wounds, post-traumatic ulcers, varicose and postphlebitic ulcers from venous blood stains, radionecroses, skin lesions, skin grafts and skin lesions due to herpes simplex. For these wound healing treatments, the HYALASTINE preparation, or the sodium salt thereof, can be administered in various methods, such as by gauze pads, cream, spray or ampoules for intradermal injectioon. For the topical applications as a cream or on a gauze pad, the HYALASTINE is preferably combined with an emulsifying agent, which absorbs the exudate from the exposed area while affording excellent diffusion of hyaluronic acid, and a water-dispersible excipient so that the wound dressing is easily removed.

The HYALECTIN fraction has been found to be particularly useful for the treatment of horses, particularly race horses, suffering from joint disorders and diseases caused by acute or chronic trauma, infections or repeated intraarticular corticosteroid injections. Specific examples of disorders treatable with HYALECTIN are osteoarthroses with or without inflammatory signs, acute or chronic synoritis, degenerative processes in articular cartilage, and dry joint disease. The most frequent symptoms of these disorders are generally pain, impaired joint function, and reduced joint flexion. The HYALECTIN fraction of the invention has been found to promote marked reduction of the healing time for such affected horses as compared with conventional therapy, to promote early and lasting improvement of joint function and to reduce pain and lameness. These clinically advantageous effects are believed to be promoted by a normalization of the viscoelasticity of the synovial fluid and by activation of tissue repair processes in the articular cartilages.

Moreover, all the above advantageous effects are promoted by the HYALECTIN fraction in the absence of local and/or systemic toxic effects. Repeated administration of HYALECTIN produces no evidence of allergic reactions nor any adverse or lasting effects.

## Pharmaceutical Preparations

The above disclosure has shown that the HYALASTINE and HYALECTIN fractions have good activity for pharmaceutical applications. The following examples are presented for exemplary purposes only to describe possible pharmaceutical preparations for actual *in vivo* administration of the HA fractions.

A. Preparations for Wound Healing

Example 1: ampoules for intradermal injection - each ampoule contains:

| | | | |
|---|---|---|---|
| -Hyalastine sodium salt | | mg | 4 |
| -Sodium Chloride | | mg | 16 |
| -Water for injection | q.s.a. | ml | 2 |

Example 2: ampoules for intradermal injection - each ampoule contains:

| | | | |
|---|---|---|---|
| -Hyalastine potassium salt | | mg | 5 |
| -Sodium Chloride | | mg | 8 |
| -Water for injection | q.s.a. | ml | 1 |

Example 3: spray-bottle for topical application - each bottle contains:

| | | | |
|---|---|---|---|
| -Hyalastine sodium salt | | mg | 20 |
| -Sodium Chloride | | mg | 80 |
| -Water for injection | q.s.a. | ml | 10 |

Example 4: spray-bottle for topical application - each bottle contains:

| | | | |
|---|---|---|---|
| -Hyalastine potassium salt | | mg | 30 |
| -Mannitol | | mg | 100 |
| -Water for injection | q.s.a. | ml | 10 |

Example 5: cream for topical application - each tube of cream contains:

| | | | |
|---|---|---|---|
| -Hyalastine potassium salt | | mg | 25 |
| -Polyethyleneglycol monostearate 400 | | mg | 1000 |
| -Cetiol (decyl ester of oleic acid) | | mg | 500 |
| -Lanette SX (cetyl-stearyl alcohol + lauryl sulfate 9:1) | | mg | 150 |
| -Glycerol | | mg | 200 |
| -Sorbitol | | mg | 150 |
| -Na-dehydroacetate | | mg | 10 |
| -p-oxymethylbenzoate | | mg | 7.5 |
| -p-oxypropylbenzoate | | mg | 5 |
| -redistilled water | q.s.a. | g | 10 |

Example 6: cream for topical application - each tube of cream contains:

| | | | |
|---|---|---|---|
| -Hyalastine sodium salt | | mg | 30 |
| -Paraffin jelly | | mg | 3 |
| -Polyethyleneglycol monostearate 400 | | mg | 1000 |
| -Cetiol (decyl ester of oleic acid) | | mg | 500 |
| -Lanette SX (cetyl-stearyl alcohol + lauryl sulfate 9:1) | | mg | 150 |
| -Glycerol | | mg | 200 |
| -Sorbitol | | mg | 150 |
| -Na-dehydroacetate | | mg | 10 |
| -p-oxymethylbenzoate | | mg | 7.5 |
| -p-oxypropylbenzoate | | mg | 5 |
| -redistilled water | q.s.a. | g | 10 |

Example 7: medicated gauze pads for topical application - each gauze-pad measuring 10x10 cm contains:

| | | | |
|---|---|---|---|
| -Hyalastine sodium salt | | mg | 3 |
| -Glycerol | | g | 1 |
| -Polyethyleneglycol | | g | 2 |
| -redistilled water | q.s.a. | g | 3 |

Example 8: medicated gauze-pads for topical application - each gauze-pad measuring 15x15 cm contains:

| | | | |
|---|---|---|---|
| -Hyalastine potassium salt | | mg | 6 |
| -Paraffin jelly | | mg | 0.5 |
| -Glycerol | | g | 1 |
| -Polyethyleneglycol | | g | 2 |
| -redistilled water | q.s.a. | g | 3 |

Example 9: dry powder for wound healing application - each gram of dry powder contains:

| | | | |
|---|---|---|---|
| -Hyalastine sodium salt | | mg | 10 |
| -Mannitol | | g | 0.75 |
| -Glycine | | g | 0.24 |

B. Preparations for Intraocular Use

Example 10: 1-ml vials - each vial contains:

| | | | |
|---|---|---|---|
| -Hyalectin sodium salt | | mg | 10 |
| -Sodium Chloride | | mg | 8 |
| -Monobasic sodium phosphate $2H_2O$ | | mg | 0.25 |
| -Dibasic sodium phosphate $12H_2O$ | | mg | 3 |
| -Water for injection | q.s.a. | ml | 1 |

Example 11: 5-ml vials - each vial contains:

| | | | |
|---|---|---|---|
| -Hyalectin potassium salt | | mg | 60 |
| -Mannitol | | mg | 50 |
| -Monobasic sodium phosphate $2H_2O$ | | mg | 1.25 |
| -Dibasic sodium phosphate $12H_2O$ | | mg | 15 |
| -water for injection | q.s.a. | ml | 5 |

Example 12: preloaded syringes - each syringe contains:

| | | | |
|---|---|---|---|
| -Hyalectin sodium salt | | mg | 40 |
| -Sodium Chloride | | mg | 16 |
| -Monobasic sodium phosphate $2H_2O$ | | mg | 0.8 |
| -Dibasic sodium phosphate $12H_2O$ | | mg | 8.16 |
| -Water for injection | q.s.a. | ml | 2 |

C. Preparations for Intra-Articular Use

Example 13: 2-ml vials - each vial contains:

| | | | |
|---|---|---|---|
| -Hyalectin sodium salt | | mg | 40 |
| -Sodium Chloride | | mg | 16 |
| -Water for injection | q.s.a. | ml | 2 |

Example 14: 4-ml vials - each vial contains:

| | | | |
|---|---|---|---|
| -Hyalectin potassium salt | | mg | 60 |
| -Mannitol | | mg | 35 |
| -Glycine | | mg | 10 |
| -Water for Injection | q.s.a. | ml | 4 |

Example 15: preloaded syringes - each syringe contains:

| | | | |
|---|---|---|---|
| -Hyalectin sodium salt | | mg | 25 |
| -Sodium Chloride | | mg | 12 |
| -Mannitol | | mg | 10 |
| -Monobasic sodium phosphate $2H_2O$ | | mg | 0.5 |
| -Dibasic sodium phosphate $12H_2O$ | | mg | 6 |
| -Water for injection | q.s.a. | ml | 2 |

Although the above-preparations have been described for exemplary purposes, it will be appreciated that other pharmaceutical formulations could be prepared by combining the HYALASTINE and HYALECTIN fractions discovered by the inventors, or the potassium or sodium salts thereof, with other pharmaceutically acceptable carriers, diluents, or excipients and at various dosages depending upon the particular use for the formulation.

For wound healing uses, the preparation of the HYALASTINE fraction are applied to the affected skin areas in one of the dosage forms discussed above, that is, either as a cream, a spray, on a gauze-pad, as a dry powder or as an intradermal injection.

For intraarticular uses, the preparations of the HYALECTIN are generally administered at a dosage rate of 2 ml per joint taken either from a prepared vial or a preloaded syringe as described above.

In an additional study, the capacity and efficiency of hyaluronic acid as a vehicle for various molecules has been investigated, in particular, use as a vehicle for ophthalmic drugs, guaranteeing perfect tolerability and compatibility (i.e., absence of sensitization phenomena) with the corneal epithelium. Hyaluronic acid is considered of particular interest as an ophthalmic vehicle. As discussed previously, HA is a glycosaminoglycan present in various connective tissues and biological liquids (such as the synovial fluid and in particular the vitreous humor) where, because of its chemical and physical nature and its striking visco-elastic characteristics, it plays a structural and biological role of fundamental importance.

Thus, a study was conducted to investigate the use of HA fractions having different molecular weights, particularly the hyalastine and hyalectin fractions, and a mixture of the same, for the preparation of different pharmaceutical forms, such as collyrium, gel, cream, inserts or dry powders. Therefore, ophthalmic drugs of different types were studied in order to obtain a broad understanding of the potential use of this biological polymer in its different fractions as a vehicle.

The experiments reported hereafter were aimed at determining whether formulations containing hyaluronic acid as an excipient are capable of enhancing the bioavailability of vehicled drugs or producing a synergistic effect in combination with the vehicled drugs, particularly with drugs having ophthalmic activity or utility.

These potential capacities of HA as a vehicle were investigated in the rabbit eye with four ophthalmic drugs of different types and actions, in particular, pilocarpine nitrate, triamcinolone, epidermal growth factor (EGF) and an antibiotic such as streptomycin and gentamicine. All of these drugs are known to have miotic, anti-inflammatory, healing and anti-microbial efficiency. Moreover evaluation of the activity of the antibiotic streptomycin, vehicled in hyaluronic acid, is very important because it is one of the most widely used antibiotics in ocular infections.

The experimental models studied and the experiments effected were as follows:

1) miotic activity of pilocarpine nitrate, vehicled in hyaluronic acid in rabbit eye;

2) anti-inflammatory activity of triamcinolone, vehicled in hyaluronic acid in the model of dextran-induced inflammation in rabbit eye;

3) healing activity of epidermal growth factor (EGF) vehicled in hyaluronic acid in a model of epithelial lesion of rabbit cornea.

4) antimicrobial activity of streptomycin vehicled in hyaluronic acid against *Bacillus subtilis* 6633 in agar plates.

*I. Miotic Activity of Pilocarpine Nitrate Vehicled In Hyaluronic Acid*

Materials

The following materials were used as excipients of pilocarpine for the various pilocarpine nitrate formulations:

hyaluronic acid sodium salt, hyalastine fraction, (m.w., approximately 100,000), at a concentration of 10 mg/ml and 20 mg/ml;

hyaluronic acid sodium salt, hyalectin fraction (m.w. 500,000—730,000), at a concentration of 10 mg/ml and 20 mg/ml;

5% polyvinyl alcohol as a comparative ophthalmic excipient.

Various 2% formulations (collyrium or gel) of pilocarpine nitrate were prepared and vehicled by adding the two different fractions of HA sodium salt at a concentration of 10 and 20 mg/ml. The following solutions were prepared:

Formulation 1 — saline with pilocarpine nitrate ($PiNO_3$) (2%), used as reference.
Formulation 2 — solution of $PiNO_3$ (2%) vehicled in 5% polyvinyl alcohol (used as reference).
Formulation 3 — solution of $PiNO_3$ (2%) vehicled in hyalastine fraction sodium salt (10 mg/ml).
Formulation 4 — solution of $PiNO_3$ (2%) vehicled in hyalastine fraction sodium salt (20 mg/ml)
Formulation 5 — solution of $PiNO_3$ (2%) vehicled in hyalectin fraction sodium salt (10 mg/ml).
Formulation 6 — solution of $PiNO_3$ (2%) vehicled in hyalectin fraction sodium salt (20 mg/ml).

Method

Albino New Zealand rabbits were used (2—2.5 kg). The formulation to be tested was instilled in one eye by microsyringe (10 l); the other eye served as a reference. The diameter of the pupil was measured in all cases at suitable time intervals. Each solution was tested on at least 8 rabbits. Each eye was treated not more than three times and a rest period of at least a week was observed between each treatment.

Parameters Measured

The pupil diameters were measured at various intervals in order to determine the miotic activity curve in time. The following activity parameters were subsequently calculated from the miosis/time graphs:

$I_{max}$ = maximum difference in pupil diameter between the treated eye and the reference.
Peak time = time taken to reach $I_{max}$.
Duration = time taken to restore basal conditions. Plateau = period of absolute miotic activity.
AUC = area under miosis/time curve.

Results

The results of the tests are reported in Table 5. It can be seen from the data for the various parameters determined from the miotic activity time curve for all the solutions tested that the addition of hyaluronic acid to a 2% pilocarpine nitrate solution gives rise to an increase in miotic activity of the drug. In fact, the bioavailability of the drug may be as much as 2.7 times greater than that of the aqueous solution containing 2% pilocarpine nitrate (Formulation 1).

It should be noted that there is a statistically significant increase in activity when the hyalectin fraction of hyaluronic acid both at 10 and 20 mg/ml is used as a vehicle (Formulations 5—6), in contrast to the pilocarpine nitrate solution vehicled in polyvinyl alcohol (Formulation 2).

The use of hyaluronic acid as a vehicle is particularly interesting because the miotic activity of pilocarpine nitrate lasts longer when it is vehicled with this substance. That is, for the hyaluronic acid containing formulations the time taken to restore pupil diameter to basal conditions is up to 190 minutes (Formulation 6) as compared to 110 minutes for pilocarpine in sale alone (Formulation 1).

*II. Anti-Inflammatory Activity Of Triamcinolone Vehicled in Hyaluronic Acid.*

Materials

The following were used:

solution of hyaluronic acid sodium salt-hyalectin fraction, m.w. between 500,000 and 730,000, 10 mg/ml in saline;

solution of triamcinolone (10% in saline).

Method

The experiments were carried out on male New Zealand rabbits (average weight 1.6 kg). After an adaptation period of 5 days, intraocular inflammation was induced in the rabbits by intraocular injection of dextran (10%, 0.1 ml). The administration was effected in both eyes under local anaesthetic with 4% Novesina, inserting the needle of the syringe by 4 mm in to the anterior chamber, at a distance of 2 mm from the limbus of the cornea. The test was conducted on 10 animals.

Table 5: Biological Activity Of Ophthalmic Formulations Containing Pilocarpine-Nitrate Vehicled By Hyaluronic Acid[a]

| Formulation | Vehicle | $I_{max}$, mm (± LF 95%) | Peak time (minutes) | Duration (minutes) | Plateau (minutes) | AUC, $cm^2$ (± LF 95%) | Relative AUC |
|---|---|---|---|---|---|---|---|
| 1 | saline | 1.93 ± 0.35 | 20 | 110 | - | 117 ± 28 | 1 |
| 2 | 5% polyvinyl alcohol | 2.33 ± 0.28 | 20 | 140 | - | 192 ± 32 | 1.64 |
| 3 | hyalastine (10 mg/ml) | 2.50 ± 0.42 | 20 | 120 | - | 240 ± 40 | 2.05 |
| 4 | hyalastine (20 mg/ml) | 2.58 ± 0.38 | 30 | 150 | - | 208 ± 41 | 1.78 |
| 5 | hyalectin (10 mg/ml) | 2.50 ± 0.38 | 15 | 170 | - | 242 ± 48 | 2.06 |
| 6 | hyalectin (20 mg/ml) | 2.70 ± 0.38 | 20 | 190 | 45 | 320 ± 45 | 2.73 |

[a] Reported values represent a mean value for 8 runs.

Treatment

Treatment was effected in each animal both in the right and left eyes, by instillation of 3 drops 3 times a day for 6 days in all, of the following:

a solution of triamcinolone (10% in saline) in the left eye (LE);

a solution of hyaluronic acid sodium salt, hyalectin fraction (10 mg/ml) + triamcinolone (10%) in the right eye (RE).

Parameters

The anti-inflammatory effect on the phlogistic reaction induced by dextran was evaluated by observing the eye with a slit lamp at the following intervals: 0, 1 hr, 3 hr, 4 hr, 48 hr, 3 days, 4 days, 5 days and 6 days.

At the intervals, the eye examination evaluated the following observations:

the state of the cornea and conjunctiva for the possible presence of hypermia, edema, and especially observation of the iris which is normally sensitive to phlogistic processes after intraocular injection of inflammatory agents;

the Tyndall effect, in which the presence of more or less intense opacity ("nubecula") is indicative of the presence of corpusculating (inflammatory) elements in the anterior chamber.

The result of the observation was expressed in terms of subjective scoring (from 0 to 3) related to the gradualness of the effect noted.

Table 6

EFFECT OF THE COMBINATION OF HYALURONIC ACID AND TRIAMCINOLONE ON INTRAOCULAR INFLAMMATION INDUCED BY DEXTRAN

Observation Interval

| | 0 | | 1hour | | 3hours | | 24hours | | 48hours | | 3days | | 4days | | 5days | | 6days | |
|---|---|---|---|---|---|---|---|---|---|---|---|---|---|---|---|---|---|---|
| | Evaluated Score[a] | | | | | | | | | | | | | | | | | |
| | LE | RE | LE | RE | LE | RE | LE | RE | LE | RE | LE | RE | LE | RE | LE | RE | LE | RE |
| Conjunctiva | 0.0 | 0.0 | 0.2 | 0.0 | 0.0 | 0.0 | 0.0 | 0.0 | 0.0 | 0.0 | 0.0 | 0.0 | 0.0 | 0.0 | 0.0 | 0.0 | 0.0 | 0.0 |
| Cornea | 0.0 | 0.0 | 1.0 | 0.2 | 0.0 | 0.7 | 0.1 | 0.0 | 0.0 | 0.0 | 0.0 | 0.0 | 0.0 | 0.0 | 0.0 | 0.0 | 0.0 | 0.0 |
| Tyndall | 0.0 | 0.0 | 1.0 | 1.2 | 3.0 | 3.0 | 3.0 | 2.1 | 3.0 | 1.2 | 3.0 | 0.2 | 2.2 | 0.0 | 1.2 | 0.0 | 0.4 | 0.0 |
| Iris | 0.0 | 0.0 | 0.5 | 0.7 | 2.7 | 2.7 | 3.0 | 2.5 | 3.0 | 1.2 | 3.0 | 0.4 | 2.4 | 0.0 | 1.5 | 0.0 | 0.5 | 0.0 |

LE = Left eye, treated with triamcinolone.

RE = Right eye, treated with tiamcinolone and hyalectin.

[a]Each value is the mean of seven observations in a total of seven animals, and expressed in terms of subjective scoring between 0 and 3, in relation to the gradualness of the effect observed.

Results

It can be seen from the results as reported in Table 6 that administration of triamcinolone has an anti-inflammatory effect on the iris and causes the disappearance of opacity (Tyndall effect) in the anterior chamber. The inflammatory process which is evident from the 1st—3rd hour until the 3rd to 4th day progressively decreases until almost normal conditions are restored, with perfect limpidness of the eye by the 6th day. On the other hand, administration of hyaluronic acid sodium salt, hyalectin fraction, together with triamcinolone reduces intraocular inflammation observed at the times discussed above relating to administration of triamcinoline alone. That is, the phlogistic process in the iris and the opacity in the anterior chamber is seen to have decreased by the 24th hour, with progressive reduction at 48 hours and with total absence of inflammatory reaction from the 4th day on.

In the conjunctiva and the cornea, essentially no notable reactions were observed after intraocular injection of dextran.

Thus, the administration of triamcinolone, together with the hyaluronic acid fraction, resulted in enhanced activity of the drug as evidence by the more rapid recovery of the rabbit eye.

*III. Healing Activity of EGF Vehicled in Hyaluronic Acid*

Materials

The following were used:

Formulation A — EGF (Epidermal growth factor), dissolved in saline (0.5 mg/5 ml)

Formulation B — hyaluronic acid sodium salt, hyalastine fraction (m.w. approximately 100,000) dissolved in saline (10 mg/ml).

Method

The experiments were carried out on male albino New Zealand rabbits (average weight 1.8 kg). The animals, after a period of adaptation of 5 days, underwent epithelial lesion of the cornea in suitable conditions of local anaesthetic with Novesina (4%). The lesion consisted of a monocular scarification of a circular area in the optic zone effected by a concave glass cylinder (ϕ 3 mm) with a sharp edge.

Treatment

The animals were subdivided into groups, each group consisting of 5 animals, and subjected to pharmacological treatment by conjunctival instillation as follows:

| Group | Treatment |
|---|---|
| Group 1 (control) | Saline |
| Group 2 | EGF solution (Formulation A) |
| Group 3 | Solution of hyaluronic acid sodium salt, hyalastine fraction, + EGF solution — combination of Formulation A + Formulation B at 1:1 ratio to make Formulation C |

Treatment was effected on the right eye (RE) by conjunctival instillation of 2 drops every 8 hours for 3 total administrations.

Parameters

Healing of the corneal epithelium was evaluated by observation of the eye and photographic documentation with a slit lamp at various intervals after scarification: 0, 8 hours, 16 hours, 24 hours, 32 hours, 40 hours, and 48 hours.

Results

Ophthalmological examination 1 as reported in Table 7, revealed that in the controls (Group 1) complete healing was achieved (5/5 animals) 48 hours after lesion. In the animals treated with EGF (Group 2) the healing process was apparent as early as 24 hours after scarification with considerable efficacy (4/5 animals). In the animals treated with the Formulation C comprising hyaluronic acid sodium salt, hyalasline fraction, + EGF (Group 3) the healing process was complete in all the animals (5/5) as early as 16 hours after scarification.

These results show that utilization of the hyalastine hyaluronic acid fraction as a vehicle for EGF enhances the healing process by promoting more rapid effective healing of corneal lesions.

Table 7

HEALING OF LESIONS IN THE CORNEAL EPITHELIUM

| Group | Treatment | Hours after scarification 0 | 8 | 16 | 24 | 48 |
|---|---|---|---|---|---|---|
| 1 | Saline | + | + | + | + | - |
| | | + | + | + | + | - |
| | | + | + | + | + | - |
| | | + | + | + | + | - |
| | | + | + | + | + | - |
| 2 | EGF (Formulation A) | + | + | + | - | - |
| | | + | + | + | - | - |
| | | + | + | + | - | - |
| | | + | + | + | + | - |
| | | + | + | + | - | - |
| 3 | Hyaluronic acid + EGF (Formulation C) | + | + | - | - | - |
| | | + | + | - | - | - |
| | | + | + | - | - | - |
| | | + | + | - | - | - |
| | | + | + | - | - | - |

\+ = unhealed eye

\- = healed eye

*IV. Antimicrobial Activity of Gentamicine Vehicled in Hyaluronic Acid*

Materials

The following materials were used:

Gentamicine dissolved in saline (50 mg/ml)

Hyaluronic acid sodium salt, hyalectin fraction (2 mg/ml).

Method

Septic phlogosis was caused in both eyes of 11 rabbits by intraocular injectioni of a titered suspension of *Pseudomonas aeruginosa* (0.1 ml). In those rabbits showing septic phlogosis, hyaluronic acid, hyalectin fraction, in combination with gentamicine was administered by instillation in the right eye and gentamicine in a buffered saline vehicle was administered in the left eye. The treatment (3 drops every 6 hours) was begun immediately after injection of the infecting agent and was continued until the infection had disappeared. The rabbits' eyes were observed every day under a slit lamp.

Results

Treatment with a combination of gentamicine with hyaluronic acid resulted in a faster disappearance of the septic infection as compared to administration of the antibiotic alone. This conclusion is clear from the data presented in Table 8.

Table 8

EFFECT OF GENTAMICINE VEHICLED IN HYALURONIC ACID HYALECTINE FRACTION, ON INTRAOCULAR SEPTIC PHLOGOSIS

| Treatment | Days from Start of Phlogosis | | | | | | |
|---|---|---|---|---|---|---|---|
| | 1 | 2 | 3 | 4 | 5 | 6 | 7 |
| Gentamicine + Buffer Saline Vehicle | 0.0 | 0.0 | 0.0 | 0.0 | 0.0 | 36.3 | 100 |
| Gentamicine + Hyaluronic Acid Hyalectine Fraction | 0.0 | 0.0 | 9.0+ | 27.2+ | 72.7+ | 100 | 100 |

Values are expressed as percentages (number of eyes cured of phlogosis by the number of eyes treated).

+ = Significant difference vs buffer vehicle (less than 0.05, Fisher exact T-Test)

Additional samples, although not limitive, of ophthalmic drugs which can be vehicled with HA fractions according to the invention are as follows:

| | |
|---|---|
| antibiotics: | chloramphenicol<br>neomycin<br>aureomycin<br>myxin and polymyxin<br>bacitracin<br>mycetins |
| hormones: | nandrolone and nandrolone sulfate |
| anesthetics (local): | henoxinate and the hydrochloride thereof |
| antiviral: | iododeoxyuridine<br>iododeoxycytidine |
| anti-inflammatories: | dexamethasone and the phosphate thereof |
| vasopressors and vasocontrictors: | synephrine and neosynephrine |

Conclusions

On the basis of the results obtained from the experiments discussed above, it can be concluded that solutions of hyaluronic acid sodium salt (in both the hyalastine and hyalectin fractions) can be used as a vehicle for ophthalmic drugs and proves to be efficient as such for various types of drugs having differing biological actions. For example, drugs including anti-glaucoma agents such as pilocarpine nitrate, anti-allergic and anti-inflammatory agents such as triamcinolone, tissue healing and cell proliferation promoting agents for promoting healing of eye tissue such as EGF, and antibiotics such as streptomycin and gentamicine, whose miotic, anti-inflammatory, healing and antimicrobial activities respectively are reported, can all be administered effectively utilizing HA as a vehicle.

The formulations of opthalmic drugs vehicled in hyaluronic acid fractions with various molecular weights prove to be perfectly tolerated by the host, and compatible with the corneal epithelium without, therefore, giving rise to sensitization phenomena.

It is also possible from the data to observe how this biological product, hyaluronic acid, is an efficient vehicle, capable of enhancing the in vivo bioavailability of vehicled drugs, strengthening the pharmacological activity of such drugs. The use of hyaluronic acid fractions as a drug vehicle particularly results in:

an increase in the miotic activity of pilocarpine nitrate while prolonging the activity time of the drug;

an increase in the anti-inflammatory activity of triamcinolone on intraocular inflammation induced by dextran, with a regression of the phlogistic process in shorter times as compared to those obtained with triamcinolone alone;

an increase in the protective action of epidermal growth factor (EGF) on superficial lesions in the cornea with obvious synergism and reduction in healing times as compared to recovery times with EGF alone; and

an increase in the in vivo biological activity of antibiotics, such as gentamicine.

The results obtained by using this biological polymer, hyaluronic acid, as a vehicle for drugs with such varied natures and actions, allows for the extrapolation of its potential as a vehicle for numerous other ophthalmic drugs.

Reported below are various examples of different formulations of ophthalmic drugs which may be in the form of powders, collyrium, gel, cream, or inserts, in which hyaluronic acid in its different fractions, hyalastine and hyalectin, is the only excipient used:

Example 1

a collyrium which may be used as "artificial tears", containing:

| | |
|---|---|
| hyaluronic acid sodium salt hyalectin fraction | 10 mg |
| saline buffered with phosphate pH 7.6 M | 10 ml |

Example 2

a collyrium which may be used as "artificial tears", containing:

| | |
|---|---|
| hyaluronic acid sodium salt hyalectin fraction | 20 mg |
| saline buffered with phosphate pH 7.6 M | 10 ml |

Example 3

a gel containing EGF in which 100 g contain:

| | |
|---|---|
| hyaluronic acid sodium salt hyalastine fraction | 55 g |
| hyaluronic acid sodium salt hyalectin fraction | 30 g |
| EGF | 0.5 g |
| twice distilled water | 23.5 g |

Example 4

a 100 mg insert with pilocarpine nitrate containing:

| | |
|---|---|
| hyaluronic acid sodium salt hyalastine fraction | 100 mg |
| pilocarpine nitrate | 2 mg |

Example 5

a powder form for topical application containing streptomycin. 100 g of powder contain:

| | |
|---|---|
| hyaluronic acid sodium salt hyalastine fraction | 70 g |
| hyaluronic acid sodium salt hyalectin fraction | 28.5 g |
| streptomycin | 1.5 g |

Although the above-preparations have been described for exemplary purposes, it will be appreciated that the pharmaceutical formulations could be prepared by combining the hyaluronic fractions, particularly the hyalectin or hyalastine fractions or the combined hyalectin/hyalastine fraction, or the potassium or sodium salts thereof, with other active drugs, and at various dosages depending upon the particular use for the formulation.

Hyaluronic acid, particularly in the substantially pure hyalectin or hyalastine fractions, has, therefore, been shown to be an effective vehicle or excipient for use in combination with various drugs having ophthalmic utility or activity. Pharmaceutical compositions containing the HA fractions as the drug vehicle are particularly useful because the HA fractions exhibit a high level of tolerability to the eye and a high compactibility with the corneal epithelium.

Use of the HA fractions, moreover, provides a means for actually enhancing the in vivo biological activity of ophthalmic drugs. The use of the particular hyalectin or hyalastine HA fractions is further useful and important because these fractions, when administered in the eye, do not exhibit undesirable inflammatory side reactions.

**Claims for the Contracting States: DE GB IT NL SE**

1. A process for preparing a substantially pure, non-inflammatory, hyaluronic acid fraction comprising: subjecting starting material tissue to solvent extraction to produce a mixture containing hyaluronic acid, and subjecting the resulting mixture to molecular filtration to obtain a hyaluronic acid fraction having an average molecular weight of from 50,000 to 730,000, said fraction being substantially free of hyaluronic acid having a molecular weight of less than 30,000.

2. A process according to Claim 1, wherein the mixture of hyaluronic acid obtained from solvent extraction is subsequently subjected to enzymatic digestion.

3. A process according to Claim 2, wherein the enzymatic digestion is conducted with papain.

4. A process according to Claim, 1, 2 or 3, wherein said mixture of hyaluronic acid is subjected to molecular filtration, with a membrane having a molecular weight exclusion limit of 30,000 to exclude molecules having a molecular weight of greater than 30,000 and thereby retain on said membrane a first hyaluronic acid fraction which is substantially free of hyaluronic acid having a molecular weight less than 30,000.

5. A process according to Claim 4, wherein said first hyaluronic acid fraction has an average molecular weight of from 250,000 to 35,000.

6. A process according to Claim 5, wherein said first hyaluronic acid fraction is subjected to further molecular ultra-filtration with a membrane having a molecular weight exclusion limit of 200,000 to exclude molecules having a molecular weight of greater than 200,000, continuing ultra-filtration until the volume of the mixture being subjected to ultra-filtration is reduced to 10% of the initial volume, and collecting the mixture which passes through the membrane to thereby obtain a second hyaluronic fraction product having an average molecular weight of from 50,000 to 100,000.

7. A process according to Claim 5, comprising collecting the mixture retained on the membrane after the ultra-filtration step with a membrane having a molecular weight exclusion limit of about 200,000 in Claim 4, thereby obtaining a third hyaluronic acid fraction product having an average molecular weight of from 500,000 to 730,000.

8. A hyaluronic acid fraction having an average molecular weight of from 50,000 to 730,000 and being substantially free of hyaluronic acid having a molecular weight of less than 30,000, or the potassium or sodium salt thereof.

9. A hyaluronic acid fraction as claimed in Claim 8, having an average molecular weight of from 250,000 to 350,000 or the potassium or sodium salt thereof.

10. A hyaluronic acid fraction as claimed in Claim 8, having an average molecular weight of from 50,000 to 100,000 or the potassium or sodium salt thereof.

11. A hyaluronic acid fraction as claimed in Claim 8, having an average molecular weight of from about 500,000 to about 730,000 or the potassium or sodium salt thereof.

12. A pharmaceutical composition containing an effective wound healing amount of the hyaluronic acid fraction according to Claim 9, and at least one pharmaceutically acceptable carrier, excipient, or diluent.

13. A pharmaceutical composition containing an effective wound healing amount of a hyaluronic acid fraction according to Claim 10, and at least one pharmaceutically acceptable carrier, diluent, or excipient.

14. A pharmaceutical composition containing an effective intraocular or intraarticular treatment amount of a hyaluronic acid fraction according to Claim 11, and at least one pharmaceutically acceptable carrier, excipient, or diluent.

15. A pharmaceutical composition for ophthalmic administration comprising an effective amount of a drug having ophthalmic activity as an active ingredient and a pharmaceutically acceptable carrier, vehicle or diluent comprising a substantially pure fraction of hyaluronic acid as defined in claim 8, or a salt thereof.

16. A pharmaceutical composition as claimed in Claim 15, wherein said hyaluronic acid fraction has an average molecular weight of from 50,000 to 100,000, from 250,000 to 350,000, or from 500,000 to 730,000, the said hyaluronic acid fraction being substantially free of hyaluronic acid having a molecular weight of less than 30,000.

17. A pharmaceutical composition as claimed in Claim 16, wherein said hyaluronic acid fraction has an average molecular weight of from 500,000 to 730,000.

18. A composition as claimed in Claim 15, 16 or 17, wherein said drug is selected from pilocarpine nitrate, triamcinolone, epidermal growth factor, streptomycin and gentamicine.

19. A substantially pure, non-inflammatory hyaluronic acid fraction having an average weight of from 50,000 to 730,000, or the potassium or sodium thereof, for use as a medicament, the fraction being substantially free of hyaluronic acid having a molecular weight of less than 30,000.

20. A substantially pure, non-inflammatory hyaluronic acid fraction having an average molecular weight of from 250,000 to 350,000, and being substantially free of hyaluronic acid having a molecular weight of less than 30,000, or the potassium or sodium salt thereof, for use as a medicament.

21. A substantially pure, non-inflammatory hyaluronic acid fraction having an average molecular weight of from 50,000 to 100,000, and being substantially free of hyaluronic acid having a molecular weight of less than 30,000, or the potassium or sodium salt thereof, for use as a medicament.

22. A substantially pure, non-inflammatory hyaluronic acid fraction having an average molecular weight of from 500,000 to 730,000, and being substantially free of hyaluronic acid having a molecular weight of less than 30,000, or the potassium or sodium salt thereof, for use in a medicament.

23. A hyaluronic acid fraction as claimed in Claim 21, for use in the healing of tissue wounds.

24. A hyaluronic acid fraction as claimed in Claim 22, for use in treating traumatic and degenerative diseases of the joints, and for improving joint function.

25. A hyaluronic acid fraction as claimed in Claim 22, for use in intraocular treatment.

26. A substantially pure fraction of hyaluronic acid as defined in claim 19, or a salt thereof for use in treating ophthalmic conditions, the said hyaluronic acid fraction acting as a vehicle for an ophthalmological drug.

27. A hyaluronic acid fraction as claimed in Claim 26 having an average molecular weight of 50,000 to 100,000, from 250,000 to 350,000, or from 500,000 to 730,000, and being substantially free of hyaluronic acid having a molecular weight of less than 30,000.

28. A hyaluronic acid fraction as claimed in Claim 27 having an average molecular weight of 500,000 to 730,000.

**Claims for the Contracting State: AT**

1. A process for preparing a substantially pure, non-inflammatory, hyaluronic acid fraction comprising: subjecting starting material tissue to solvent extraction to produce a mixture containing hyaluronic acid, and subjecting the resulting mixture to molecular filtration to obtain a hyaluronic acid fraction having an average molecular weight of from 50,000 to 730,000, said fraction being substantially free of hyaluronic acid having a molecular weight of less than 30,000.

2. A process according to Claim 1, wherein the mixture of hyaluronic acid obtained from solvent extraction is subsequently subjected to enzymatic digestion.

3. A process according to Claim 2, wherein the enzymatic digestion is conducted with papain.

4. A process according to Claim, 1, 2 or 3, wherein said mixture of hyaluronic acid is subjected to molecular filtration with a membrane having a molecular weight exclusion limit of 30,000 to exclude molecules having a molecular weight of greater than 30,000 and thereby retain on said membrane a first hyaluronic acid fraction which is substantially free of hyaluronic acid having a molecular weight less than 30,000.

5. A process according to Claim 4, wherein said first hyaluronic acid fraction has an average molecular weight of from 250,000 to 350,000.

6. A process according to Claim 5, wherein said first hyaluronic acid fraction is subjected to further molecular ultra-filtration with a membrane having a molecular weight exclusion limit of 200,000 to exclude molecules having a molecular weight of greater than 200,000, continuing ultra-filtration until the volume of the mixture being subjected to ultra-filtration is reduced to 10% of the initial volume, and collecting the mixture which passes through the membrane to thereby obtain a second hyaluronic fraction product having an average molecular weight of from 50,000 to 100,000.

7. A process according to Claim 5, comprising collecting the mixture retained on the membrane after the ultra-filtration step with a membrane having a molecular weight exclusion limit of about 200,000 in Claim 4, thereby obtaining a third hyaluronic acid fraction product having an average molecular weight of from 500,000 to 730,000.

8. A process as claimed in any one of the preceding claims, wherein the hyaluronic acid fractions are in the form of the potassium or sodium salt thereof.

9. A process as claimed in any one of the preceding claims, wherein the hyaluronic acid fractions or salt thereof is for use as a medicament.

10. A process according to Claim 9, wherein the hyaluronic acid fraction is substantially pure and non-inflammatory and has an average molecular weight of from about 50,000 to about 730,000.

11. A process according to Claim 9, wherein the hyaluronic acid fraction is substantially pure and non-inflammatory and has an average molecular weight of from about 250,000 to about 350,000.

12. A process according to Claim 9, wherein the hyaluronic acid fraction is substantially pure and non-inflammatory and has an average molecular weight of from about 50,000 to about 100,000.

13. A process according to Claim 9, wherein the hyaluronic acid fraction is substantially pure and non-inflammatory and has an average molecular weight of from about 500,000 to about 730,000.

14. A process according to Claim 12, wherein the hyaluronic acid fraction or salt thereof is for use in the healing of tissue wounds.

15. A process according to Claim 13, wherein the hyaluronic acid fraction or a salt thereof is for use in treating traumatic and degenerative diseases of the joints, and for improving joint function.

16. A process according to Claim 13, wherein the hyaluronic acid fraction or a salt thereof is for use in intraocular treatment.

17. A process according to anyone of Claims 1 to 8, wherein the hyaluronic acid fraction, or salt thereof, is for use in treating ophthalmic conditions, the said hyaluronic acid fraction acting as a vehicle for an ophthalmological drug.

18. A process for preparing a pharmaceutical composition, the method comprising the steps of providing an effective wound healing amount of the hyaluronic acid fraction which is prepared by the method according to Claim 5 and combining that fraction with at least one pharmaceutically acceptable carrier, excipient, or diluent.

19. A process for preparing a pharmaceutical composition, the method comprising the steps of providing an effective wound healing amount of the hyaluronic acid fraction which is prepared by the method according to Claim 6 and combining that fraction with at least one pharmaceutically acceptable carrier, diluent, or excipient.

20. A process for preparing a pharmaceutical composition, the method comprising the steps of providing an effective intraocular or intraarticular treatment amount of a hyaluronic acid fraction which is prepared by the method according to Claim 7 and combining that fraction with at least one pharmaceutically acceptable carrier, excipient, or diluent.

21. A process for preparing a pharmaceutical composition for ophthalmic administration, the method comprising combining an effective amount of a drug having ophthalmic activity as an active ingredient and a pharmaceutically acceptable carrier, vehicle or diluent comprising a substantially pure fraction of hyaluronic acid as defined in claim 10, or a salt thereof.

22. A process as claimed in Claim 21, wherein said hyaluronic acid fraction has an average molecular weight of from about 50,000 to about 100,000, from about 250,000 to about 350,000, or from about 500,000 to 730,000, said hyaluronic acid fraction being substantially free of hyaluronic acid, or a salt thereof, having a molecular weight of less than 30,000.

23. A process as claimed in Claim 21, wherein said hyaluronic acid fraction has an average molecular weight of from about 500,000 to about 730,000, said hyaluronic acid fraction being substantially free of hyaluronic acid, or a salt thereof, having a molecular weight of less than 30,000.

24. A process as claimed in Claim 21, 22 or 23, wherein said drug is selected from pilocarpine nitrate, triamcinolone, epidermal growth factor, streptomycin and gentamicine.

**Patentansprüche für die Vertragsstaaten: DE GB IT NL SE**

1. Verfahren zur Herstellung einer im wesentlichen reinen, keine Entzündungen hervorrufenden Hyaluronsäure-Fraktion, welches darin besteht, daß man das Ausgangsmaterial-Gewebe einer Lösungsmittel-Extraktion unterwirft, um ein Hyaluronsäure enthaltendes Gemisch zu produzieren, das resultierende Gemisch einer Molekülfiltration unterwirft, un eine Hyaluronsäure-Fraktion mit einem durchschnittlichen Molekulargewicht von 50 000 bis 730 000 zu erhalten, wobei diese Fraktion im wesentlichen frei ist von Hyaluronsäure mit einem Molekulargewicht von weniger als 30 000.

2. Verfahren nach Anspruch 1, worin das aus der Lösungsmittel-Extraktion erhaltene Gemisch von Hyaluronsäure anschließend enzymatischer Verdauung unterworfen wird.

3. Verfahren nach Anspruch 2, worin die enzymatische Verdauung mit Papain durchgeführt wird.

4. Verfahren nach Anspruch 1 oder 3, worin das Gemisch von Hyaluronsäure der Molekülfiltration mit einer Membran mit einer Molekulargewicht-Ausschlußgrenze von 30 000 unterworfen wird, um Moleküle mit einem Molekulargewicht von mehr als 30 000 auszuschließen un dabei auf besagter Membran eine erste Hyaluronsäure-Fraktion zurückzuhalten, die im wesentlichen frei von Hyaluronsäure mit einem Molekulargewicht von weniger als 30 000 ist.

5. Verfahren nach Anspruch 4, worin diese erste Hyaluronsäure-Fraktion ein durchschnittliches Molekulargewicht von 250 000 bis 350 000 ist.

6. Verfahren nach Anspruch 5, worin besagte erste Hyaluronsäure-Fraktion weiterer Molekülutrafiltration mit einer Membran mit einer Molekulargewicht-Ausschlußgrenze von 200 000 unterworfen wird, um Moleküle auszuschließen, die ein Molekulargewicht von mehr als 200 000 haben, wobei die Ultrafiltration fortgesetzt wird, bis das Volumen des Gemischs, das der Ultrafiltration unterworfen wird, auf 10% des Anfangsvolumens vermindert ist, und das Gemisch, das durch die Membran geht, gesammelt wird, um dadurch ein zweites Hyaluronsäure-Fraktionsprodukt zu erhalten, das ein durchschnittliches Molekulargewicht von 50 000 bis 100 000 hat.

7. Vefahren nach Anspruch 5, umfassend das Sammeln des auf der Membran nach dem Ultrafiltrationsschritt mit einer Membran mit einer Molekulargewicht-Ausschlußgrenze von etwa 200 000 im Anspruch 4 zurückgehaltenen Gemischs, wobei ein drittes Hyaluronsäure-Fraktionsprodukt erhalten wird, das ein durchschnittliches Molekulargewicht von 500 000 bis 730 000 hat.

8. Hyaluronsäure-Fraktion mit einem durchschnittlichen Molekulargewicht von 50 000 bis 730 000, im wesentlichen frei von Hyaluronsäure mit einem Molekulargewicht von weniger als 30 000, oder das Kaluim- oder Natriumsalz davon.

9. Hyaluronsäure-Fraktion nach Anspruch 8 mit einem durchschnittlichen Molekulargewicht von 250 000 bis 350 000 oder das Kalium- oder Natriumsalz davon.

10. Hyaluronsäure-Fraktion nach Anspruch 8 mit einem durchschnittlichen Molekulargewicht von 50 000 bis 100 000 oder das Kalium- oder Natriumsalz davon.

11. Hyaluronsäure-Fraktion nach Anspruch 8 mit einem durchschnittlichen Molekulargewicht von etwa 500 000 bis etwa 730 000 oder das Kalium- oder Natriumsalz davon.

12. Pharmazeutische Zusammensetzung, die eine wirksame wundheilende Menge der Hyaluronsäure-Fraktion nach Anspruch 9 und wenigstens einen pharmazeutisch annehmbaren Träger, Exzipienten oder ein Verdünnungsmittel enthält.

13. Pharazeutische Zusammensetzung, die eine wirksame wundheilende Menge einer Hyaluronsäure-Fraktion nach Anspruch 10 und wenigstens einen pharmazeutisch annehmbaren Träger, ein Verdünnungsmittel oder einen Exzipienten enthält.

14. Pharmazeutisch Zusammensetzung, die eine intraokuläre oder intraartikuläre Behandlung wirksame Menge einer Hyaluronsäure-Fraktion nach Anspruch 11 und wenigstens einen pharmazeutisch annehmbaren Träger, Exzipienten oder ein Verdünnungsmittel enthält.

15. Pharmazeutische Zusammensetzung zur ophthalmischen Anwendung, umfassend eine wirksame Menge eines Medikaments mit ophthalmischer Aktivität als Wirkstoff und einen pharmazeutisch annehmbaren Träger, ein Vehikel oder Verdünnungsmittel, enthaltend eine im wesentlichen reine Fraktion von Hyaluronsäure, wie im Anspruch 8 definiert, oder ein Salz davon.

16. Pharmazeutische Zusammensetung nach Anspruch 15, worin besagte Hyaluronsäure-Fraktion ein durchschnittliches Molekulargewicht von 50 000 bis 100 000 von 250 000 bis 350 000 oder von 500 000 bis 730 000 hat und besagte Hyaluronsäure-Fraktion im wesentlichen frei ist von Hyaluronsäure mit einem Molekulargewicht von weniger als 30 000.

17. Pharmazeutische Zusammensetzung nach Anspruch 16, worin besagte Hyaluronsäure-Fraktion ein durchschnittliches Molekulargewicht von 500 000 bis 730 000 hat.

18. Zusammensetzung nach Anspruch 15, 16 oder 17, worin besagtes Medikament ausgewählt wird aus Pilocarpin-nitrat, Triamcinolon, epidermalem Wachstumsfaktor, Streptomycin und Gentamicin.

19. Im wesentlichen reine, keine Entzündungen hervorrufende Hyaluronsäure-Fraktion mit einem durchschnittlichen Molekulargewicht von 50 000 bis 730 000, oder das Kalium- oder Natriumsalz davon zur Verwendung als Medikament, wobei die Fraktion im wesentlichen frei ist von Hyaluronsäure mit einem Molekulargewicht von weniger als 30 000.

20. Im wesentlichen reine, keine Entzündungen hervorrufende Hyaluronsäure-Fraktion mit einem durchsnittlichen Molekulargewicht von 250 000 bis 350 000, im wesentlichen frei von Hyaluronsäure mit einem Molekulargewicht von weniger als 30 000, oder das Kalium- oder Natriumsalz davon zur Verwendung als Medikament.

21. Im wesentlichen reine, keine Entzündungen hervorrufende Hyaluronsäure-Fraktion mit einem durchschnittlichen Molekulargewicht von 50 000 bis 100 000, im wesentlichen frei von Hyaluronsäure mit einem Molekulargewicht von weniger als 30 000, oder das Kalium- oder Natriumsalz davon zur Verwendung als Medikament.

22. Im wesentlichen reine, keine Entzündungen hervorrufende Hyaluronsäure-Fraktion mit einem durchschnittlichen Molekulargewicht von 500 000 bis 730 000, im wesentlichen frei von Hyaluronsäure mit einem Molekulargewicht von weniger als 30 000, oder das Kalium- oder Natriumsalz davon zur Verwendung als Medikament.

23. Hyaluronsäure-Fraktion nach Anspruch 21 zur Verwendung beim Heilen von Gewebewunden.

24. Hyaluronsäure-Fraktion nach Anspruch 22 zur Verwendung bei der Behandlung traumatischer und degenerativer Erkrankungen der Gelenke und zur Verbesserung der Gelenksfunktion.

25. Hyaluronsäure-Fraktion nach Anspruch 22 zur Verwendung bei der intrakulären Behandlung.

26. Im wesentlichen reine Fraktion von Hyaluronsäure, wie im Anspruch 19 definiert, oder ein Salz davon zur Verwendung bei der Behandlung ophthalmischer Zustände, wobei besagte Hyaluronsäure-Fraktion also Vehikel für ein ophthalmologisches Medikament fungiert.

27. Hyaluronsäure-Fraktion nach Anspruch 26 mit einem durchschnittlichen Molekulargewicht von 50 000 bis 100 000, von 250 000 bis 350 000 oder von 500 000 bis 730 000, im wesentlichen frei von Hyaluronsäure mit einem Molekulargewicht von weniger als 30 000.

28. Hyaluronsäure-Fraktion nach Anspruch 27 mit einem durchschnittlichen Molekulargewicht von 500 000 bis 730 000.

**Patentansprüche für den Vertragsstaat: AT**

1. Verfahren zur Herstellung einer im wesentlichen reinen, keine Entzündungen hervorrufenden Hyaluronsäure-Fraktion, welches darin besteht, daß man das Ausgangsmaterial-Gewebe einer Lösungsmittel-Extraktion unterwirft, um ein Hyaluronsäure enthaltendes Gemisch zu produzieren, das resultierende Gemisch einer Molekülfiltration unterwirft, un eine Hyaluronsäure-Fraktion mit einem durchschnittlichen Molekulargewicht von 50 000 bis 730 000 zu erhalten, wobei diese Fraktion im wesentlichen frei ist von Hyaluronsäure mit einem Molekulargewicht von weniger als 30 000.

2. Verfahren nach Anspruch 1, worin das aus der Lösungsmittel-Extraktion erhaltene Gemisch von Hyaluronsäure anschließend enzymatischer Verdauung unterworfen wird.

3. Verfahren nach Anspruch 2, worin die enzymatische Verdauung mit Papain durchgeführt wird.

4. Verfahren nach Anspruch 1 oder 3, worin das Gemisch von Hyaluronsäure der Molekülfiltration mit einer Membran mit einer Molekulargewicht-Ausschlußgrenze von 30 000 unterworfen wird, um Moleküle mit einem Molekulargewicht von mehr als 30 000 auszuschließen und dabei auf besagter Membran eine erste Hyaluronsäure-Fraktion zurückzuhalten, die im wesentlichen frei von Hyaluronsäure mit einem Molekulargewicht von weniger als 30 000 ist.

5. Verfahren nach Anspruch 4, worin diese erste Hyaluronsäure-Fraktion ein durchschnittliches Molekulargewicht von 250 000 bis 350 000 hat.

6. Verfahren nach Anspruch 5, worin besagte erste Hyaluronsäure-Fraktion weiterer Molekülutrafiltration mit einer Membran mit einer Molekulargewicht-Ausschlußgrenze von 200 000 unterworfen wird, um Moleküle auszuschließen, die ein Molekulargewicht von mehr als 200 000 haben, wobei die Ultrafiltration fortgesetzt wird, bis das Volumen des Gemischs, das der Ultrafiltration unterworfen wird, auf 10% des Anfangsvolumens vermindert ist, und das Gemisch, das durch die Membran geht, gesammelt wird, um dadurch ein zweites Hyaluronsäure-Fraktionsprodukt zu erhalten, das ein durchschnittliches Molekulargewicht von 50 000 bis 100 000 hat.

7. Vefahren nach Anspruch 5, umfassend das Sammeln des auf der Membran nach dem Ultrafiltrationsschritt mit einer Membran mit einer Molekulargewicht-Ausschlußgrenze von etwa 200 000 im Anspruch 4 zurückgehaltenen Gemischs, wobei ein drittes Hyaluronsäure-Fraktionsprodukt erhalten wird, das ein durchschnittliches Molekulargewicht von 500 000 bis 730 000 hat.

8. Verfahren nach einen der vorstehend genannten Ansprüche, worin die Hyaluronsäure-Fraktionen in form des Kalium- oder Natriumsalzes davon vorliegen.

9. Verfahren nach einem der vorstehend genannten Ansprüche, worin die Hyaluronsäure-Fraktionen oder Salz davon als Medikament verwendet werden.

10. Verfahren nach Anspruch 9, worin die Hyaluronsäure-Fraktion im wesentlichen rein ist und keine Entzündungen hervorruft und ein durchschnittliches Molekulargewicht von etwa 50 000 bis eta 730 000 hat.

11. Verfahren nach Anspruch 9, worin die Hyaluronsäure-Fraktion im wesentlichen rein ist und keine Entzündungen hervorruft und ein durchschnittliches Molekulargewicht von etwa 250 000 bis etwa 350 000 hat.

12 Verfahren nach Anspruch 9, worin die Hyaluronsäure-Fraktion im wesentlichen rein ist und keine Entzündungen hervorruft un eine durchschnittliches Molekulargewicht von etwa 50 000 bis etwa 100 000 hat.

13. Verfahren nach Anspruch 9, worin die Hyaluronsäure-Fraktion im wesentlichen rein ist und keine Entzündungen hervorruft und ein durchschnittliches Molekulargewicht von etwa 500 000 bis etwa 730 000 hat.

14. Verfahren nach Anspruch 12, worin die Hyaluronsäure-Fraktion oder das Salz davon zur Verwendung bei der Heilung von Gewebewunden dient.

15. Verfahren nach Anspruch 13, worin die Hyaluronsäure-Fraktion oder ein Salz davon zur Verwendung bei der Behandlung traumatischer und degenerativer Erkrankungen der Gelenke und zur Verbesserung der Gelenksfunktion dient.

16. Verfahren nach Anspruch 13, worin die Hyaluronsäure-Fraktion oder ein Salz davon zur Verwendung bei der intraokulären Behandlung dient.

17. Verfahren nach einem der Ansprüche 1 bis 8, worin die Hyaluronsäure-Fraktion oder ein Salz davon der Behandlung ophthalmischer Zustände dient, wobei besagte Hyaluronsäure-Fraktion als Vehikel für ein ophthalmologisches Medikament dient.

18. Verfahren zur Herstellung einer pharmazeutischen Zusammensetzung, wobei das Verfahren das Bereitstellen einer wirksamen wundheilenden Menge der Hyaluronsäure-Fraktion, die nach dem Verfahren von Anspruch 5 hergestellt wird, und das Vereinigen dieser Fraktion mit wenigstens einem pharmazeutisch annehmbaren Träger, Exzipienten oder Verdünnungsmittel umfaßt.

19. Verfahren zur Herstellung einer pharmazeutischen Zusammensetzung, wobei das Verfahren die Schritte Bereitstellen einer wirksamen wundheilenden Menge der Hyaluronsäure-Fraktion, die nach dem Verfahren nach Anspruch 6 hergestellt wird, und Vereinigen dieser Fraktion mit wenigstens einem pharmazeutisch annehmbaren Träger, Verdünnungsmittel oder Exzipienten umfaßt.

20. Verfahren zur Herstellung einer pharmazeutischen Zusammensetzung, wobei das Verfahren die Schritte Bereitstellen einer für intraokuläre oder intraartikuläre Behandlung wirksamen Menge einer Hyaluronsäure-Fraktion, die nach dem Verfahren nach Anspruch 7 hergestellt wurde, und Vereinigen dieser Fraktion mit wenigstens einem pharmazeutisch annehmbaren Träger, Exzipienten oder Verdünnungsmittel umfaßt.

21. Verfahren zur Herstellung einer pharmazeutischen Zusammensetzung zur ophthalmischen Anwendung, welches Verfahren das Vereinigen einer wirksamen Menge eines Medikaments mit ophthalmischer Aktivität als Wirkstoff und eines pharmazeutisch annehmbaren Trägers, Vehikels oder Verdünnungsmittels umfaßt, enthaltend eine im wesentlichen reine Fraktion von Hyaluronsäure, wie im Anspruch 10 definiert, oder ein Salz davon.

22. Verfahren nach Anspruch 21, worin die Hyaluronsäure-Fraktion ein durchshnittliches Molekulargewicht von etwa 50 000 bis etwa 100 000, von etwa 250 000 bis etwa 350 000 oder von etwa 500 000 bis etwa 730 000 hat, die Hyaluronsäure-Fraktion im wesentlichen frei ist von Hyaluronsäure oder einem Salze davon mit einem Molekulargewicht von weniger also 30 000.

23. Verfahren nach Anspruch 21, worin die Hyaluronsäure-Fraktion ein durchschnittliches Molekulargewicht von etwa 500 000 bis etwa 730 000 hat, die Hyaluronsäure-Fraktion im wesentlichen frei ist von Hyaluronsäure oder einem Salz davon mit einem Molekulargewicht von weniger als 30 000.

24. Verfahren nach Anspruch 21, 22 oder 23, worin das Medikament ausgewählt wird aus Pilocarpinnitrat, Triamcinolon, epidermalem Wachstumsfaktor, Streptomycin und Gentamicin.

**Revendications pour les Etats contractants: DE GB IT NL SE**

1. Procédé pour la préparation d'une fraction d'acide hyaluronique non-inflammatoire, pratiquement pure, consistant à soumettre un tissu servant de matière première à une extraction aux solvants, pour produire un mélange contenant de l'acide hyaluronique, et à soumettre le mélange résultant à une filtration moléculaire pour obtenir une fraction d'acide hyaluronique ayant un masse moléculaire moyenne de 50 000 à 730 000, ladite fraction étant pratiquement exempte d'acide hyaluronique ayant une masse moléculaire inférieure à 30 000.

2. Procédé selon la revendication 1, dans lequel le mélange d'acide hyaluronique obtenu à partir de l'extraction aux solvants est ensuite soumis à une digestion enzymatique.

3. Procédé selon la revendication 2, dans lequel la digestion enzymatique est réalisée avec de la papaïne.

4. Procédé selon la revendication 1, 2 ou 3, dans lequel ledit mélange d'acide hyaluronique est soumis à une filtration moléculaire avec une membrane ayant une limite d'exclusion de masse moléculaire de 30 000, pour exclure les molécules ayant une masse moléculaire supérieure à 30 000 et ainsi retenir sur ladite membrane une première fraction d'acide hyaluronique pratiquement exempte d'acide hyaluronique ayant une masse moléculaire inférieure à 30 000.

5. Procédé selon la revendication 4, dans lequel ladite première fraction d'acide hyaluronique a une masse moléculaire moyenne de 250 000 à 350 000.

6. Procédé selon la revendication 5, dans lequel ladite première fraction d'acide hyaluronique est soumise à une ultrafiltration moléculaire plus poussée avec une membrane ayant une limite d'exclusion de masse moléculaire de 200 000, pour exclure les molécules ayant une masse moléculaire supérieure à 200 000, à une ultrafiltration continue jusqu'à ce que le volume du mélange soumis à l'ultrafiltration soit réduit à 10% du volume initial, et collecte du mélange qui traverse la membrane, pour obtenir un produit constitué d'une deuxième fraction d'acide hyaluronique ayant une masse moléculaire moyenne de 50 000 à 100 000.

7. Procédé selon la revendication 5, consistant à recueillir le mélange retenu sur la membrane après l'étape d'ultrafiltration à l'aide d'une membrane ayant une limite d'exclusion de masse moléculaire d'environ 200 000 dans la revendication 4, pour obtenir un troisième produit constitué d'une fraction d'acide hyaluronique ayant une masse moléculaire moyenne de 500 000 à 730 000.

8. Fraction d'acide hyaluronique ayant une masse moléculaire moyenne de 50 000 à 730 000, et pratiquement exempte d'acide hyaluronique ayant une masse moléculaire inférieure à 30 000, ou son sel de potassium ou de sodium.

9. Fraction d'acide hyaluronique selon la revendication 8, ayant une masse moléculaire moyenne de 250 000 à 350 000, ou son sel de potassium ou de sodium.

10. Fraction d'acide hyaluronique selon la revendication 8, ayant un masse moléculaire moyenne de 50 000 à 100 000, ou son sel de potassium ou de sodium.

11. Fraction d'acide hyaluronique selon la revendication 8, ayant un masse moléculaire moyenne d'environ 500 000 à environ 730 000, ou son sel de potassium ou de sodium.

12. Composition pharmaceutique contentant un quantité cicatrisante efficace de la fraction d'acide hyaluronique selon la revendication 9, et au moins un support, excipient ou diluant pharmaceutiquement acceptable.

13. Composition pharmaceutique contenant une quantité cicatrisante efficace de la fraction d'acide hyaluronique selon la revendication 10, et au moins un support, excipient ou diluant pharmaceutiquement acceptable.

14. Composition pharmaceutique contentant une quantité efficace pour un traitement intra-oculaire ou intra-articulaire d'une fraction d'acide hyaluronique selon la revendication 11 et au moins un support, excipient ou diluant pharmaceutiquement acceptable.

15. Composition pharmaceutique pour administration ophthalmique, comprenant en tant que principe actif une quantité efficace d'une substance médicamenteuse ayant une activité ophthalmique, et un support, véhicule ou diluant pharmaceutiquement acceptable, comprenant une fraction pratiquement pure d'acide hyaluronique comme défini dans la revendication 8, ou l'une de ses sels.

16. Composition pharmaceutique selon la revendication 15, dans lequelle fraction d'acide hyaluronique a une masse moléculaire moyenne de 50 000 à 100 000, de 250 000 à 350 000 ou de 500 000 à 730 000, ladite fraction d'acide hyaluronique étant pratiquement exempte d'acide hyaluronique ayant une masse moléculaire inférieure à 30 000.

17. Composition pharmaceutique selon la revendication 16, dans laquelle ladite fraction d'acide hyaluronique a une masse moléculaire moyenne de 500 000 à 730 000.

18. Composition selon la revendication 15, 16 ou 17, dans laquelle ladite substance médicamenteuse est choisie parmi le nitrate de pilocarpine, la triamcinolone, le facteur de croissance épidermique, la streptomycine et la gentamicine.

19. Fraction d'acide hyaluronique non-inflammatoire, pratiquement pure, ayant une masse moléculaire moyenne de 50 000 à 730 000, ou son sel de potassium ou de sodium, pour utilisation comme médicament, la fraction étant pratiquement exempte d'acide hyaluronique ayant une masse moléculaire inférieure à 30 000.

20. Fraction d'acide hyaluronique non-inflammatoire, pratiquement pure, ayant une masse moléculaire moyenne de 250 000 à 350 000, et pratiquement exempte d'acide hyaluronique ayant une masse moléculaire inférieure à 30 000, ou son sel de potassium ou de sodium, pour utilisation comme médicament.

21. Fraction d'acide hyaluronique non-inflammatoire, pratiquement pure, ayant une masse moléculaire moyenne de 50 000 à 100 000, et pratiquement exempte d'acide hyaluronique ayant une masse moléculaire inférieure à 30 000, ou son sel de potassium ou de sodium, pour utilisation comme médicament.

22. Fraction d'acide hyaluronique non-inflammatoire, pratiquement pure, ayant une masse moléculaire moyenne de 500 000 à 730 000, et pratiquement exempte d'acide hyaluronique ayant une masse moléculaire inférieure à 30 000, ou son sel de potassium ou de sodium, pour utilisation comme médicament.

23. Fraction d'acide hyaluronique selon la revendication 1, pour la cicatrisation des tissus.

24. Fraction d'acide hyaluronique selon la revendication 22, pour utilisation dans le traitement des lésions traumatiques et dégénératives de articulations, et pour améliorer le fonctionnement des articulations.

25. Fraction d'acide hyaluronique selon la revendication 22, pour utilisation en traitement intra-oculaire.

26. Fraction pratiquement pure d'acide hyaluronique selon la revendication 19, ou l'une de ses sels, pour utilisation dans le traitement des conditions ophthalmiques, ladite fraction d'acide hyaluronique jouant le rôle d'un véhicule pour le médicament ophtalmologique.

27. Fraction d'acide hyaluronique selon la revendication 26, ayant une masse moléculaire moyenne de 50 000 à 100 000, de 250 000 à 350 000 ou de 500 000 à 730 000, et pratiquement exempte d'acide hyaluronique ayant une masse moléculaire inférieure à 30 000.

28. Fraction d'acide hyaluronique selon la revendication 27, ayant une masse moléculaire moyenne de 500 000 à 730 000.

**Revendications pour l'Etat contractant: AT**

1. Procédé pour la préparation d'une fraction d'acide hyaluronique non-inflammatoire, pratiquement pure, consistant à soumettre un tissu servant de matière première à une extraction aux solvants, pour produire un mélange contenant de l'acide hyaluronique, et à soumettre le mélange résultant à une filtration moléculaire pour obtenir une fraction d'acide hyaluronique ayant un masse moléculaire moyenne de 50 000 à 730 000, ladite fraction étant pratiquement exempte d'acide hyaluronique ayant une masse moléculaire inférieure à 30 000.

2. Procédé selon la revendication 1, dans lequel le mélange d'acide hyaluronique obtenu à partir de l'extraction aux solvants est ensuite soumis à une digestion enzymatique.

3. Procédé selon la revendication 2, dans lequel la digestion enzymatique est réalisée avec de la papaïne.

4. Procédé selon la revendication 1, 2 ou 3, dans lequel ledit mélange d'acide hyaluronique est soumis à une filtration moléculaire avec une membrane ayant une limite d'exclusion de masse moléculaire de 30 000, pour exclure les molécules ayant une masse moléculaire supérieure à 30 000 et ainsi retenir sur ladite membrane une première fraction d'acide hyaluronique pratiquement exempte d'acide hyaluronique ayant une masse moléculaire inférieure à 30 000.

5. Procédé selon la revendication 4, dans lequel ladite première fraction d'acide hyaluronique a une masse moléculaire moyenne de 250 000 à 350 000.

6. Procédé selon la revendication 5, dans lequel ladite première fraction d'acide hyaluronique est soumise à une ultrafiltration moléculaire plus poussée avec une membrane ayant une limite d'exclusion de masse moléculaire de 200 000, pour exclure les molécules ayant une masse moléculaire supérieure à 200 000, à une ultrafiltration continue jusqu'à ce que le volume du mélange soumis à l'ultrafiltration soit réduit à 10% du volume initial, et collecte du mélange qui traverse la membrane, pour obtenir un produit constitué d'une deuxième fraction d'acide hyaluronique ayant une masse moléculaire moyenne de 50 000 à 100 000.

7. Procédé selon la revendication 5, consistant à recueillir le mélange retenu sur la membrane après l'étape d'ultrafiltration à l'aide d'une membrane ayant une limite d'exclusion de masse moléculaire d'environ 200 000 dans la revendication 4, pour obtenir un troisième produit constitué d'une fraction d'acide hyaluronique ayant une masse moléculaire moyenne de 500 000 à 730 000.

8. Procédé selon l'une quelconque des revendications précédentes, dans lequel les fractions d'acide hyaluronique se présentent sous forme de leur sel de potassium ou de sodium.

9. Procédé selon l'une quelconque des revendications précédentes, dans lequel les fractions d'acide hyaluronique ou leur sel sont destinés à une utilisation comme médicament.

10. Procédé selon la revendication 9, dans lequel la fraction d'acide hyaluronique est pratiquement pure et non-inflammatoire, et a une masse moléculaire moyenne d'environ 50 000 à environ 730 000.

11. Procédé selon la revendication 9, dans lequel la fraction d'acide hyaluronique est pratiquement pure et non-inflammatoire, et a une masse moléculaire moyenne d'environ 250 000 à environ 350 000.

12. Procédé selon la revendication 9, dans lequel la fraction d'acide hyaluronique est pratiquement pure et non-inflammatoire, et a une masse moléculaire moyenne d'environ 50 000 à environ 100 000.

13. Procédé selon la revendication 9, dans lequel la fraction d'acide hyaluronique est pratiquement pure et non-inflammatoire, et a une masse moléculaire moyenne d'environ 500 000 à environ 730 000.

14. Procédé selon la revendication 12, dans lequel la fraction d'acide hyaluronique ou son sel est destinée à une utilisation pour la cicatrisation des tissus.

15. Procédé selon la revendication 13, dans lequel la fraction d'acide hyaluronique ou l'une de ses sels est destiné à être utilisé dans le traitement des lésions traumatiques et dégénératives des articulations, et pour améliorer le fonctionnement des articulations.

16. Procédé selon la revendication 13, dans lequel la fraction d'acide hyaluronique ou l'un de ses sels est destiné à être utilisé dans le traitement intra-oculaire.

17. Procédé selon l'une quelconque des revendications 1 à 8, dans laquelle le fraction d'acide hyaluronique ou son sel est destiné à être utilisé dans le traitement de conditions ophtalmiques, ladite fraction d'acide hyaluronique jouant le rôle d'un véhicule pour un médicament ophthalmologique.

18. Procédé de préparation d'une composition pharmaceutique, ce procédé comprenant les étapes consistant à mettre à disposition une quantité cicatrisante efficace de la fraction d'acide hyaluronique préparée par le procédé de la revendication 5, et à combiner cette fraction à au moins un support excipient ou diluant pharmaceutiquement acceptable.

19. Procédé de préparation d'une composition pharmaceutique, ce procédé comprenant les étapes consistant à mettre à disposition une quantité cicatrisante efficace de la fraction d'acide hyaluronique préparée par le procédé de la revendication 6, et à combiner cette fraction à au moins un support, excipient ou diluant pharmaceutiquement acceptable.

20. Procédé de préparation d'une composition pharmaceutique, ce procédé comprenant les étapes consistant à mettre à disposition une quantité cicatrisante efficace de la fraction d'acide hyaluronique préparée par le procédé de la revendication 7, et à combiner cette fraction à au moins un support, excipient ou diluant pharmaceutiquement acceptable.

21. Procédé de préparation d'une composition pharmaceutique pour administration ophtalmique, ce procédé consistant à combiner, et tant que principe actif, une quantité efficace d'une substance médicamenteuse ayant une activité ophtalmique, et un support, véhicule ou diluant pharmaceutiquement acceptable, comprenant une fraction pratiquement pure d'acide hyaluronique selon la revendication 10, ou l'un de ses sels.

22. Procédé selon la revendication 21, dans lequel la fraction d'acide hyaluronique a une masse moléculaire moyenne d'environ 50 000 à environ 100 000, d'environ 250 000 à environ 350 000 ou d'environ 500 000 à environ 730 000, ladite fraction d'acide hyaluronique étant pratiquement exempte d'acide hyaluronique, ou de l'un de ses sels, ayant une masse moléculaire inférieure à 30 000.

23. Procédé selon la revendication 21, dans lequel ladite fraction d'acide hyaluronique a une masse moléculaire moyenne d'environ 500 000 à environ 730 000, ladite fraction d'acide hyaluronique étant pratiquement exempte d'acide hyaluronique, ou de l'une de ses sels, ayant une masse moléculaire inféreiure à 30 000.

24. Procédé selon la revendication 21, 22 ou 23, dans lequel ladite substance médicamenteuse est choisie parmi le nitrate de pilocarpine, la triamcinolone, le facteur de croissance épidermique, la streptomycine et la gentamicine.

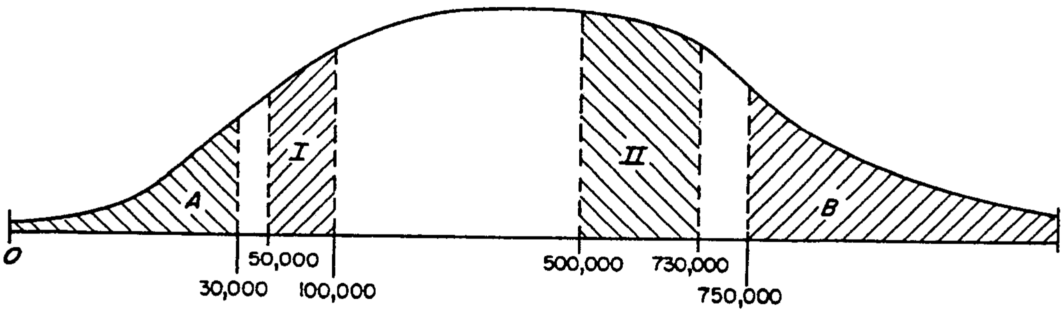


AVERAGE MOLECULAR WEIGHT OF HYALURONIC ACID